# EUROPEAN PATENT APPLICATION

(11) **EP 4 403 616 A1**
(43) Date of publication of application: **24.07.2024**
(21) Application number: 22870321.1
(22) Date of filing: 15.09.2022
(51) Int. Cl.: C12M 1/00, C12M 1/34, C12M 1/12

(54) **PORTABLE BIOREACTOR**

(30) Priority: 16.09.2021 KR 20210124294
(71) Applicant: Amogreentech Co., Ltd., Gimpo-si, Gyeonggi-do 10014 (KR)
(72) Inventor: JANG, Seon Ho, Gimpo-si, Gyeonggi-do 10014 (KR); HAN, Kyoung Ku, Gimpo-si, Gyeonggi-do 10014 (KR); SEO, In Yong, Gimpo-si, Gyeonggi-do 10014 (KR); LEE, Seoung Hoon, Gimpo-si, Gyeonggi-do 10014 (KR); SONG, Jae Kyung, Gimpo-si, Gyeonggi-do 10014 (KR); KOO, Song Hee, Gimpo-si, Gyeonggi-do 10014 (KR); PARK, Hee Sung, Gimpo-si, Gyeonggi-do 10014 (KR); NOH, Hyeong Tak, Gimpo-si, Gyeonggi-do 10014 (KR); KIM, Ji Young, Gimpo-si, Gyeonggi-do 10014 (KR); LEE, Su Yeon, Gimpo-si, Gyeonggi-do 10014 (KR); CHO, Seung Min, Gimpo-si, Gyeonggi-do 10014 (KR)
(74) Representative: Laine IP Oy
(86) International application number: PCT/KR2022/013819
(87) International publication number: WO 2023/043237

(57) **Abstract**

Provided is a portable bioreactor. The portable bioreactor according to an embodiment of the present invention may comprise: a frame unit; a cell culture unit which has one side mounted to the frame unit and in which a plurality of supports for cell culture are arranged, the cell culture unit being provided with an inlet, through which a medium supplied from the outside flows in, and an outlet, through which the medium inside the cell culture unit is discharged to the outside; a medium adjustment unit installed in the frame unit and storing therein a certain amount of medium for the cell culture; a circulation pump installed and fixed to the frame unit to circulate the medium stored in the medium adjustment unit; and a gas supply unit for supplying gas to the medium adjustment unit to maintain the medium stored in the medium adjustment unit at a constant pH

## Description

### Technical Field

The present invention relates to a portable bioreactor.

### Background Art

Animal cells derived from human or animal tissue may be cultured while suspended or attached to a scaffold in a medium. Generally, blood cell-derived cells (including hematopoietic stem cells) are suspension cells, and tissue-derived cells such as skin-derived, liver-derived, or lung-derived cells and embryonic stem cells or mesenchymal stem cells are adherent cells. The suspension cells can proliferate while suspended alone in the medium, but the adherent cells can proliferate only when attached to a surface of the scaffold.

Accordingly, since suspension cells are more advantageous in maintaining the highest cell density per unit volume in cell scale-up than adherent cells, most methods for mass culture of cells are for suspension cells, and development of methods or devices for mass culture of adherent cells is insufficient.

In addition, since the size of the entire facility of a mass cell culture system for mass culture of adherent cells is very large, there is a problem in that cell culture using the conventional mass cell culture system is only possible in a limited space in which the mass cell culture system is installed.

### Disclosure

### Technical Problem

The present invention is directed to providing a portable bioreactor that is portable and can culture a large amount of adherent cells through a single process.

### Technical Solution

The present invention provides a portable bioreactor including: a frame part; a cell culture part having one side mounted on the frame part, a plurality of scaffolds for cell culture disposed therein, an inlet through which a medium supplied from the outside is introduced, and an outlet through which the medium inside the cell culture part is discharged to the outside; a medium adjustment part installed at the frame part and configured to store therein a certain amount of medium for cell culture; a circulation pump installed to be fixed to the frame part to circulate the medium stored in the medium adjustment part; and a gas supply part configured to supply a gas to the medium adjustment part for the medium stored in the medium adjustment part to maintain a constant pH level.

Also, the frame part may include a base plate, a rack extending a predetermined height from the base plate for the cell culture part to be mounted while spaced a predetermined height from the base plate, and a mounting plate extending a predetermined height from the base plate for the medium adjustment part and the circulation pump to be mounted.

Also, the cell culture part may include a box-shaped housing having an accommodation space filled with the medium, the plurality of scaffolds arranged parallel in one direction in the accommodation space for cells to be cultured, and a spacing member configured to space two scaffolds facing each other for the plurality of scaffolds to remain spaced from each other.

Also, the scaffold may include a plate-shaped nanofiber membrane coated with a protein motif and a support member attached to one surface of the nanofiber membrane via an adhesive layer to support the nanofiber membrane.

Also, the medium adjustment part may include a box-shaped storage container having an open upper portion, a stopper configured to cover the open upper portion of the storage container, and a plurality of fittings formed at the stopper to be connected to each of the circulation pump, the inlet of the cell culture part, and the gas supply part via a connecting tube, the inside of the storage container may be, through the certain amount of medium filled therein, divided into a medium storage space filled with the medium and a gas storage space positioned at an upper side of the medium storage space and filled with air, and the medium filled in the medium storage space may maintain a constant pH level through the gas supplied from the gas supply part to the gas storage space.

Also, the medium adjustment part may further include a vent port provided at the stopper to communicate with the gas storage space to prevent generation of bubbles while the gas is supplied from the gas supply part to the gas storage space.

Also, it may include a first fitting connected to the inlet of the cell culture part via the circulation pump for the medium of the medium storage space to be supplied to the cell culture part, a second fitting connected to the outlet of the cell culture part for the medium of the cell culture part to be recovered to the medium storage space, and a third fitting connected to the gas supply part for the gas supplied from the gas supply part to be introduced into the gas storage space.

In addition, the medium adjustment part may include a first extension tube connected to the first fitting for one end of the first extension tube to be submerged in the medium stored in the medium storage space and a second extension tube connected to the second fitting for one end of the second extension tube to be submerged in the medium stored in the medium storage space.

### Advantageous Effects

According to the present invention, since a large amount of cells can be simultaneously cultured through a single process, productivity can be improved, and due to portability, cells can be cultured in various locations without limitations.

### Description of Drawings

FIG. 1 is a schematic diagram illustrating a portable bioreactor according to one embodiment of the present invention.
FIG. 2 is a view illustrating a cell culture part that may be applied to FIG. 1.
FIG. 3 is a view of a scaffold assembly illustrated in FIG. 2 and illustrates a state in which some scaffolds are separated from the scaffold assembly.
FIG. 4 is a cross-sectional view of a cell culture part in direction A-A of FIG. 1.
FIG. 5 shows cross-sectional views of scaffolds that may be applied to the portable bioreactor according to one embodiment of the present invention.
FIG. 6 is a cross-sectional view of a medium adjustment part in direction B-B of FIG. 1.

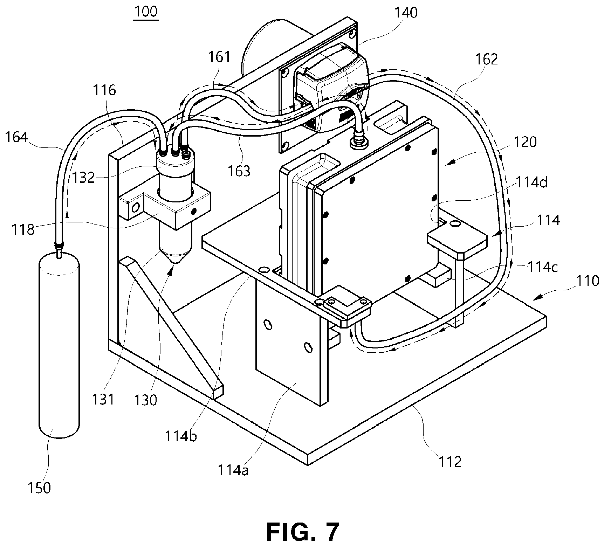

FIG. 7 is a schematic diagram illustrating a movement path of a fluid in the portable bioreactor according to one embodiment of the present invention.
FIG. 8 is a view schematically illustrating a form in which the portable bioreactor according to one embodiment of the present invention is stored in a case.

### Modes of the Invention

Hereinafter, embodiments of the present invention will be described in detail with reference to the accompanying drawings so that those of ordinary skill in the art to which the present invention pertains can easily carry out the present invention. The present invention may be implemented in various different forms and is not limited to the embodiments described herein. In the drawings, in order to clearly describe the present invention, parts irrelevant to the description are omitted, and like or similar components are denoted by like reference numerals throughout the specification.

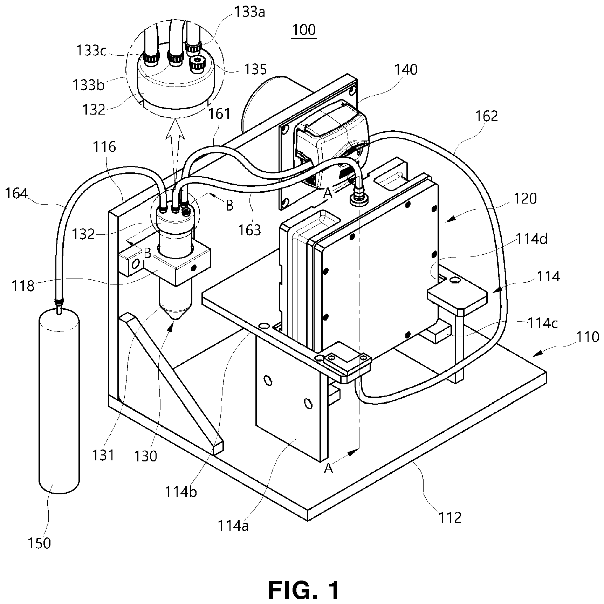

As illustrated in FIG. 1, a portable bioreactor 100 according to one embodiment of the present invention includes a frame part 110, a cell culture part 120, a medium adjustment part 130, a circulation pump 140, and a gas supply part 150.

The cell culture part 120, the medium adjustment part 130, and the circulation pump 140 may be fixed to or mounted on the frame part 110.

That is, the portable bioreactor 100 according to one embodiment of the present invention may be configured in the form of a single module by the cell culture part 120, the medium adjustment part 130, and the circulation pump 140 being fixed to or mounted on one side of the frame part 110.

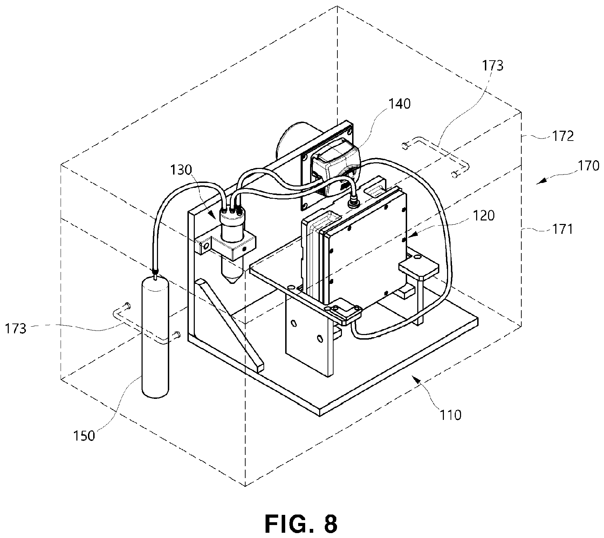

Accordingly, as illustrated in FIG. 8, the frame part 110 of the portable bioreactor 100 according to one embodiment of the present invention may be inserted into a separate case 170 in a state in which the cell culture part 120, the medium adjustment part 130, and the circulation pump 140 are fixed to or mounted on the frame part 110.

Accordingly, when the portable bioreactor 100 according to one embodiment of the present invention is inserted into the case 170, the portable bioreactor 100 according to one embodiment of the present invention may be portable and easily transported to various locations by transporting and/or moving the case 170.

As a result, since the portable bioreactor 100 according to one embodiment of the present invention can be carried or transported by a user, cells can be cultured in various locations without limitations.

Here, the case 170 may include a first case 171 in the shape of a box having one open side and a second case 172 configured to cover the open portion of the first case 171, and a handle 173 may be provided at one side of the first case 171 for the user to easily hold the case 170.

Accordingly, the user may insert the frame part 110 having the cell culture part 120, the medium adjustment part 130, and the circulation pump 140 mounted thereon into the first case 171 and then use the handle 173 to easily transport the portable bioreactor 100 according to one embodiment of the present invention.

However, the shape of the case 170 is not limited thereto and may be changed to various other known forms as long as the user can easily transport the case 170 after accommodating the frame part 110 having the cell culture part 120, the medium adjustment part 130, and the circulation pump 140 mounted thereon in the first case 171.

In addition, the overall size of the portable bioreactor 100 according to one embodiment of the present invention may be 20 cm * 30 cm * 20 cm, but the present invention is not limited thereto, and the overall size may be changed to various other sizes as long as a worker can easily transport the portable bioreactor 100 according to one embodiment of the present invention with his or her own strength without using a separate tool. That is, the overall size of the portable bioreactor 100 according to one embodiment of the present invention may be smaller than or equal to 100 cm * 100 cm * 100 cm.

As a specific example, as illustrated in FIG. 1, the frame part 110 may include a base plate 112, a rack 114, and a mounting plate 116.

For example, the base plate 112 may be a plate-shaped member having a predetermined area, and the mounting plate 116 may be a plate-shaped member having a predetermined area and extending a predetermined height from the base plate 112.

Also, the rack 114 may include a pair of vertical plates 114a extending a predetermined height from the base plate 112, a plate-shaped rack plate 114b connecting upper ends of the pair of vertical plates 114a, and a pair of supports 114c provided at surfaces of the pair of vertical plates 114a that face each other.

In such a case, the pair of supports 114c may be disposed at a lower portion of the rack plate 114b to be spaced a predetermined distance from the rack plate 114b, and the rack plate 114b may include a cutout groove 114d formed by being cut out to insert a portion of the cell culture part 120.

Accordingly, when one side of the cell culture part 120 is inserted into the rack plate 114b through the cutout groove 114d, the one side of the cell culture part 120 may be supported by the supports 114c, and an inlet 122 of the cell culture part 120 may remain spaced a predetermined height from the base plate 112.

In such a case, a holder member 118 for fixing the medium adjustment part 130 may be installed at the mounting plate 116 side, and the circulation pump 140 may be installed to be fixed to the mounting plate 116.

Accordingly, in the portable bioreactor 100 according to one embodiment of the present invention, each of the cell culture part 120, the medium adjustment part 130, and the circulation pump 140 may be mounted on the frame part 110.

As a result, the portable bioreactor 100 according to one embodiment of the present invention may be inserted into the case 170 in a state in which the cell culture part 120, the medium adjustment part 130, and the circulation pump 140 are mounted on the frame part 110 as described above, and when the frame part 110 is inserted into the case 170, the user may easily transport and move the portable bioreactor 100 according to one embodiment of the present invention by moving and transporting the case 170.

Accordingly, since the user can carry or transport the portable bioreactor 100 according to one embodiment of the present invention in a state in which the frame part 110 having the cell culture part 120, the medium adjustment part 130, and the circulation pump 140 mounted thereon is stored in the case 170, the portable bioreactor 100 may be transported to various locations without limitations in order to culture cells.

Here, the various locations may be locations that provide a culture environment in which cells attached to scaffolds 125 and 125' can be stably cultured. An example of the various locations may be a small-scale incubator provided in a laboratory, and the small-scale incubator may be a space in which a constant temperature is maintained.

The cell culture part 120 may be mounted on the frame part 110 as described above, and the plurality of scaffolds 125 and 125' for cell culture may be disposed inside the cell culture part 120.

That is, the cell culture part 120 may provide a space in which cells attached to the plurality of scaffolds 125 and 125' are cultured and may include the inlet 122 through which a medium supplied from the outside is introduced and an outlet 123 through which the medium inside the cell culture part 120 is discharged to the outside.

Here, the inlet 122 may be connected to the circulation pump 140, which is connected to the medium adjustment part 130, via a connecting tube 162, and the outlet 123 may be connected to the medium adjustment part 130 via a connecting tube 163.

In such a case, the cell culture part 120 may be mounted on the frame part 110 with the inlet 122 positioned at a lower portion and the outlet 123 positioned at an upper portion, and the plurality of scaffolds 125 and 125' may be arranged to be spaced at predetermined intervals from each other in parallel while disposed perpendicular to the base plate 112 of the frame part 110.

Accordingly, the medium supplied through the inlet 122 may move upward through a space formed between the scaffolds 125 and 125' disposed in an accommodation space S1 and then may be discharged to the outside through the outlet 123.

Here, cells to be cultured may be attached to the plurality of scaffolds 125 and 125', and the cells attached to the scaffolds 125 and 125' may receive nutrients from the medium filled in the accommodation space S1 which will be described below. Accordingly, culture of the cells attached to the plurality of scaffolds 125 and 125' may be facilitated through the nutrients supplied from the medium.

In this case, the plurality of scaffolds 125 and 125' may be provided in a plate shape having a predetermined area, and as described above, the plurality of scaffolds 125 and 125' formed in the plate shape may be arranged in the accommodation space S1 and spaced apart from other scaffolds 125 and 125' arranged parallel thereto.

As a result, in the portable bioreactor 100 according to one embodiment of the present invention, a degree of integration of the scaffolds 125 and 125' disposed in the accommodation space S1 may be increased, and thus a large amount of cells can be cultured through a single culturing process.

In addition, in the portable bioreactor 100 according to one embodiment of the present invention, since the plurality of scaffolds 125 and 125' may be arranged in parallel to each other in a single cell culture part 120, the size of the cell culture part 120 can be reduced while mass culture of cells is possible.

Accordingly, since the frame part 110 having the cell culture part 120 mounted thereon may be inserted into the case 170 as described above, the portable bioreactor 100 according to one embodiment of the present invention can culture a large amount of cells through the cell culture part 120 while being able to be carried by transporting and/or moving the case 170.

To this end, for the scaffolds 125 and 125', various known materials used in cell culture may be used without limitations as long as the scaffolds 125 and 125' can be implemented in a plate shape, and cells can be easily attached to the scaffolds 125 and 125'.

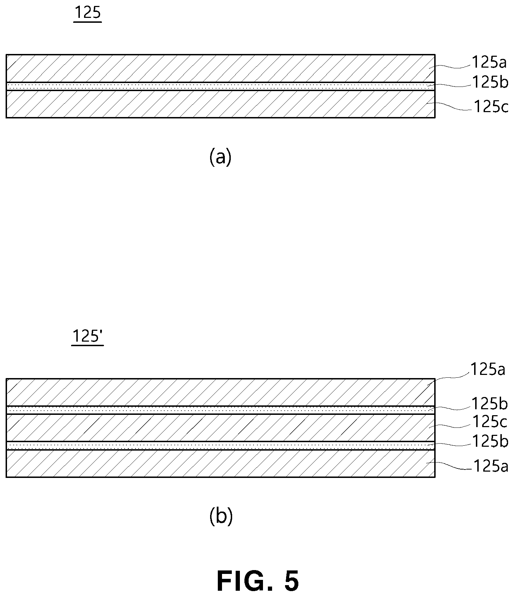

As a non-limiting example, the scaffolds 125 and 125' may include a nanofiber membrane 125a in which nanofibers are formed in a three-dimensional network structure through electrospinning. In such a case, as illustrated in FIG. 5(a), the scaffold 125 may have a three-layer structure that includes a support member 125c attached to one surface of the nanofiber membrane 125a via an adhesive layer 125b, in addition to the nanofiber membrane 125a.

Here, the support member 125c may be a plate-shaped film member and may support one surface of the nanofiber membrane 125a. Accordingly, the nanofiber membrane 125a may be supported through the support member 125c even when formed in a plate shape having flexibility, and thus bending or drooping of the nanofiber membrane 125a can be prevented. Accordingly, since the scaffold 125 disposed in the accommodation space S1 of the cell culture part 120 can maintain an unfolded state, cell culture is facilitated.

However, the structure of the scaffold 125 is not limited thereto, and as illustrated in FIG. 5(b), the scaffold 125' may have a five-layer structure in which the nanofiber membrane 125a is attached to both surfaces of the support member 125c via the adhesive layer 125b.

In addition, the scaffolds 125 and 125' may have improved surfaces to facilitate attachment of cells to be cultured. That is, the nanofiber membrane 125a may be a membrane in which surfaces of nanofibers are motif-coated. Accordingly, cells to be cultured may be smoothly attached to the surfaces of the scaffolds 125 and 125', and the cells to be cultured may be cultured through nutrients supplied from the medium while attached to the surfaces of the scaffolds 125 and 125'.

However, the type of the scaffolds 125 and 125' is not limited thereto, and various other known materials used in cell culture may be used as long as the scaffolds 125 and 125' can be implemented in a plate shape, and cells can be easily attached to the scaffolds 125 and 125'.

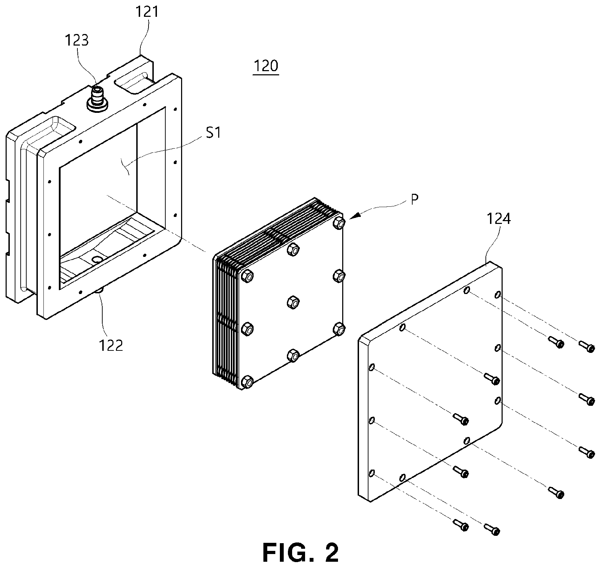

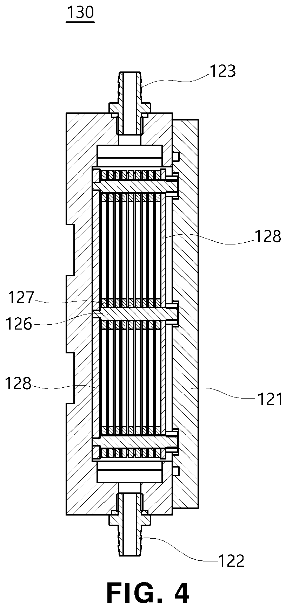

As illustrated in FIGS. 2 to 4, the cell culture part 120 may include a housing and the above-described scaffolds 125 and 125'.

The housing may have the plurality of scaffolds 125 and 125' for cell culture disposed therein and thus provide a space in which cells attached to the plurality of scaffolds 125 and 125' are cultured.

To this end, the housing may be formed in the shape of a box having the accommodation space S1. For example, as illustrated in FIG. 2, the housing may include a body 121 formed in the shape of a box having the accommodation space S1 whose one side is open, the body 121 may include the inlet 122 and the outlet 123 each formed at one side, and the accommodation space S1 with one open side may be closed through a cover 124 coupled to the body 121.

Accordingly, the medium supplied from the outside to the housing may be filled in the accommodation space S1 through the inlet 122, and the medium filled in the accommodation space S1 may be discharged to the outside through the outlet 123.

In this case, in the cell culture part 120, the plurality of scaffolds 125 and 125' may be integrated in the form of an assembly to improve assemblability while increasing the degree of integration of the plurality of scaffolds 125 and 125' to facilitate mass culture of cells.

For example, the cell culture part 120 may include a scaffold assembly P disposed to be inserted into the accommodation space S 1, and the scaffold assembly P may have a form in which the plurality of scaffolds 125 and 125' are integrated while spaced predetermined intervals from each other.

Specifically, the scaffold assembly P may be configured as a stacked type in which the plurality of scaffolds 125 and 125' are disposed parallel to and spaced from each other in a thickness direction of the body 121.

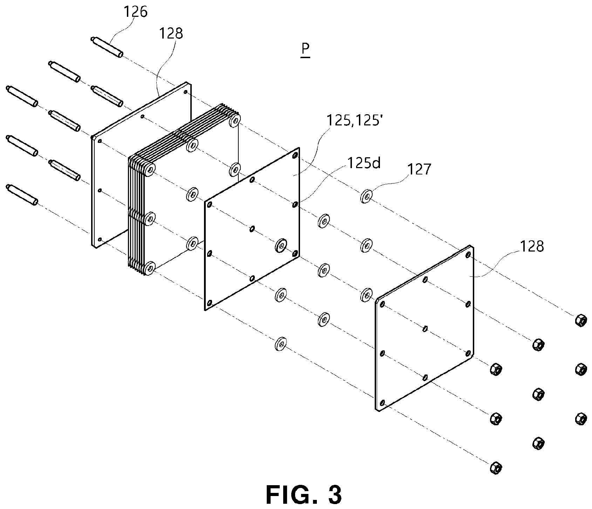

To this end, as illustrated in FIG. 3, the scaffold assembly P may include a plurality of support bars 126 having a predetermined length and a plurality of spacing members 127 formed in a ring shape, and the plurality of scaffolds 125 and 125' may be fitted to the support bars 126.

In such a case, the plurality of support bars 126 may be disposed to be spaced at predetermined intervals from each other, and the plurality of support bars 126 may have both ends each fixed to plate-shaped support plates 128 having a predetermined area.

Accordingly, the plurality of support bars 126 having both ends each fixed to one of the two support plates 128 may remain spaced predetermined intervals from each other, the plurality of scaffolds 125 and 125' may be disposed parallel to each other between the two support plates 128, and the plurality of scaffolds 125 and 125' may be fastened to the support bars 126 through a plurality of through-holes 125d formed at positions corresponding to the plurality of support bars 126.

In addition, like the scaffolds 125 and 125', each of the plurality of spacing members 127 may be fitted to one of the plurality of support bars 126, and the plurality of spacing members 127 and the plurality of scaffolds 125 and 125' may be alternately fastened to the support bars 126.

Accordingly, the spacing member 127 may be disposed between the two scaffolds 125 and 125' arranged parallel to each other, and the two scaffolds 125 and 125' neighboring each other may remain spaced from each other through the spacing member 127.

However, the scaffold assembly P is not limited thereto, and various other known methods may be applied as long as the plurality of scaffolds 125 and 125' can remain spaced certain intervals from each other while being arranged parallel to each other in one direction.

The medium adjustment part 130 may store therein a certain amount of medium containing nutrients necessary for cell culture. As described above, the medium adjustment part 130 may be mounted on the mounting plate 116 via the holder member 118 installed at the mounting plate 116, and the medium stored in the medium adjustment part 130 may be supplied to the cell culture part 120 and then be recovered from the cell culture part 120 through the circulation pump 140.

In this case, even when the medium is recovered from the cell culture part 120 toward the medium adjustment part 130 through the circulation pump 140, the medium adjustment part 130 may always supply the medium having a constant pH value toward the cell culture part 120.

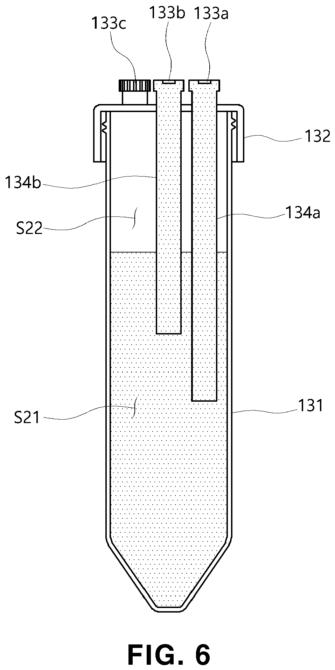

To this end, as illustrated in FIGS. 1 and 6, the medium adjustment part 130 may include a storage container 131 having an inner space for storing a certain amount of the medium and formed in the shape of a box whose upper portion is open, a stopper 132 configured to cover the open upper portion of the storage container 131, and a plurality of fittings 133a, 133b, and 133c formed at the stopper 132. In such a case, the plurality of fittings 133a, 133b, and 133c may be connected to the circulation pump 140, the outlet 123 of the cell culture part 120, and the gas supply part 150 via connecting tubes 161, 163, and 164, respectively.

Also, the inner space of the storage container 131 in which a certain amount of the medium is stored may be, through the certain amount of the medium filled therein, divided into a medium storage space S21 filled with the medium and a gas storage space S22 positioned at an upper side of the medium storage space S21 and filled with air.

In such a case, the plurality of fittings may include a first fitting 133a connected to the circulation pump 140 for the medium of the medium storage space S21 to be supplied to the cell culture part 120, a second fitting 133b connected to the outlet 123 of the cell culture part for the medium of the cell culture part 120 to be recovered to the medium storage space S21, and a third fitting 133c connected to the gas supply part 150 via a connecting tube 164 for the gas supplied from the gas supply part 150 to be introduced into the gas storage space S22.

Also, as illustrated in FIG. 6, the medium adjustment part 130 may further include a first extension tube 134a connected to the first fitting 133a and a second extension tube 134b connected to the second fitting 133b, and the first extension tube 134a and the second extension tube 134b may be disposed so that one end of each of the first extension tube 134a and the second extension tube 134b is submerged in the medium stored in the medium storage space S21.

In addition, the medium adjustment part 130 may further include a vent port 135 provided at the stopper 132 to communicate with the gas storage space S22. Here, in a case where a gas is supplied from the gas supply part 150 to the gas storage space S22, the vent port 135 may prevent generation of bubbles inside the storage container 131 due to the gas supplied from the gas supply part 150.

Accordingly, the medium stored in the medium storage space S21 of the storage container 131 may be supplied toward the cell culture part 120 through the first extension tube 134a and the first fitting 133a and then recovered to the medium storage space S21 of the storage container 131 through the second fitting 133b and the second extension tube 134b by the operation of the circulation pump 140.

In this case, the gas supply part 150 may supply a gas to the gas storage space S22 of the medium adjustment part 130, and some of the gas present in the gas storage space S22 may be discharged to the outside through the vent port 135.

Accordingly, the gas may be smoothly supplied from the gas supply part 150 toward the gas storage space S22, the gas that has moved to the gas storage space S22 may be dissolved toward the medium stored in the medium storage space S21, and the medium stored in the medium storage space S21 may maintain a certain pH level through the dissolved gas.

As a result, even when the medium used in cell culture is recovered from the cell culture part 120 to the medium storage space S21 by the operation of the circulation pump 140, the medium present in the medium storage space S21 may always maintain a pH level suitable for cell culture and be supplied toward the cell culture part 120 again.

That is, even when the concentration of the dissolved gas is decreased in a process in which the medium stored in the medium storage space S21 is moved to the cell culture part 120 and then recovered toward the medium storage space S21 through the second fitting 133b, the pH level of the medium recovered to the medium storage space S21 may be changed to an appropriate pH level necessary for cell culture through the gas supplied from the gas supply part 150.

As a result, even when the medium repeatedly circulates in the cell culture part 120 and the medium adjustment part 130 through the circulation pump 140, the cells attached to the scaffolds 125 and 125' may continuously receive the medium whose pH level is suitable for cell culture, and thus culture of the cells is facilitated.

Here, the gas supply part 150 may be a gas container configured to store a predetermined gas therein, and the gas supplied from the gas supply part 150 may be a gas mixture in which carbon dioxide, nitrogen, and oxygen are mixed at a predetermined ratio. However, the type of the gas supplied from the gas supply part 150 is not limited thereto, and the gas supplied from the gas supply part 150 may be 100% carbon dioxide gas or may be appropriately changed according to the type of cells cultured in the cell culture part 120.

In addition, a gas-permeable filter member (not illustrated) configured to allow passage of gas and block movement of foreign matter and liquid may be provided at the vent port 135 side.

In this way, since the portable bioreactor 100 according to one embodiment of the present invention is configured so that the medium adjustment part 130, the circulation pump 140, and the cell culture part 120 are connected to each other via the connecting tubes 161, 162, and 163, and the medium stored in the medium adjustment part 130 circulates in the medium adjustment part 130 and the cell culture part 120 through the circulation pump 140, a closed medium circulation system can be implemented.

In addition, in the portable bioreactor 100 according to one embodiment of the present invention, since the medium of the medium adjustment part 130 can maintain a certain pH level suitable for cell culture through the gas supplied from the gas supply part 150, the medium necessary for cell culture can be reused, thus minimizing the amount of used medium and reducing production costs.

Further, in the portable bioreactor 100 according to one embodiment of the present invention, the supply amount, supply cycle, supply time, and the like of the gas supplied from the gas supply part 150 to the medium adjustment part 130 may be controlled by a separate controller (not illustrated), and the controller may control the overall operation in addition to the operation of the circulation pump 140.

Embodiments of the present invention have been described above, but the spirit of the present invention is not limited by the embodiments presented herein, and those of ordinary skill in the art who understand the spirit of the present invention may easily propose other embodiments by adding other components, changing components, or omitting components within the scope of the same spirit. However, such embodiments also belong to the scope of the spirit of the present invention.

## Claims

1. A portable bioreactor comprising:
a frame part;
a cell culture part having one side mounted on the frame part, a plurality of scaffolds for cell culture disposed therein, an inlet through which a medium supplied from the outside is introduced, and an outlet through which the medium inside the cell culture part is discharged to the outside;
a medium adjustment part installed at the frame part and configured to store therein a certain amount of medium for cell culture;
a circulation pump installed to be fixed to the frame part for the medium stored in the medium adjustment part to circulate in the cell culture part and the medium adjustment part; and
a gas supply part configured to supply a gas to the medium adjustment part for the medium stored in the medium adjustment part to maintain a constant pH level.

2. The portable bioreactor of claim 1, wherein the frame part includes a base plate, a rack extending a predetermined height from the base plate for the cell culture part to be mounted while spaced a predetermined height from the base plate, and a mounting plate extending a predetermined height from the base plate for the medium adjustment part and the circulation pump to be mounted.

3. The portable bioreactor of claim 1, wherein the cell culture part includes a box-shaped housing having an accommodation space filled with the medium, the plurality of scaffolds arranged parallel in one direction in the accommodation space for cells to be cultured, and a spacing member configured to space two scaffolds facing each other for the plurality of scaffolds to remain spaced from each other.

4. The portable bioreactor of claim 1, wherein the scaffold includes a plate-shaped nanofiber membrane coated with a protein motif and a support member attached to one surface of the nanofiber membrane via an adhesive layer to support the nanofiber membrane.

5. The portable bioreactor of claim 1, wherein:
the medium adjustment part includes a box-shaped storage container having an open upper portion, a stopper configured to cover the open upper portion of the storage container, and a plurality of fittings formed at the stopper to be connected to each of the circulation pump, the outlet of the cell culture part, and the gas supply part via a connecting tube;
the inside of the storage container is, through the certain amount of medium filled therein, divided into a medium storage space filled with the medium and a gas storage space positioned at an upper side of the medium storage space and filled with air; and
the medium filled in the medium storage space maintains a constant pH level through the gas supplied from the gas supply part to the gas storage space.

6. The portable bioreactor of claim 5, wherein the medium adjustment part further includes a vent port provided at the stopper to communicate with the gas storage space to prevent generation of bubbles while the gas is supplied from the gas supply part to the gas storage space.

7. The portable bioreactor of claim 5, wherein the plurality of fittings include a first fitting connected to the circulation pump for the medium of the medium storage space to be supplied to the cell culture part, a second fitting connected to the outlet of the cell culture part for the medium of the cell culture part to be recovered to the medium storage space, and a third fitting connected to the gas supply part for the gas supplied from the gas supply part to be introduced into the gas storage space.

8. The portable bioreactor of claim 7, wherein the medium adjustment part includes a first extension tube connected to the first fitting for one end of the first extension tube to be submerged in the medium stored in the medium storage space and a second extension tube connected to the second fitting for one end of the second extension tube to be submerged in the medium stored in the medium storage space.
